# EUROPEAN PATENT APPLICATION

(11) **EP 2 397 071 A1**
(43) Date of publication of application: **21.12.2011**
(21) Application number: 10166283.1
(22) Date of filing: 17.06.2010
(51) Int. Cl.: A61B 5/04, A61N 1/05

(54) **Neural probe with a modular microelectrode for stimulating neurons or recording neural activity**

(30) Priority: 16.06.2010 US 355381 P
(71) Applicant: IMEC, 3001 Leuven (BE); Katholieke Universiteit Leuven, 3000 Leuven (BE)
(72) Inventor: Pereira Neves, Hercules, B-1320, Hamme-Mille (BE); Orban, Guy, B-1950, Kraainem (BE)
(74) Representative: Bird, William Edward

(57) **Abstract**

The present application relates to a neural probe system (6) comprising:
• a carrier (7),
• at least one planar microelectrode (2) attached to the carrier (7), said planar microelectrode (2) comprising a set of two or more conductive segments (1), said set being confined in an area of from 15 μm² to 8000 μm², and
• electrical connections (4,5) assuring that at least two subsets of conductive segments (1) within said set are individually switchable to a same input and/or output line (3) so that the surface area and/or the shape of the microelectrode (2) electrically connected to said line (3) can be varied.

## Description

### Technical field of the invention

The present invention relates to the field of neural probes and to their manufacture and use for exciting neurons and/or acquiring data.

### Background of the invention

Neural probes are devices comprising microelectrodes for stimulating or recording the activity of neurons. They are used in both fundamental research and medical applications. In all cases, selectivity and impedance are in a trade-off situation since higher selectivity means a smaller electrode, and a smaller electrode-neuronal tissue interface leads to higher impedance. Performing experiments at different selectivities or impedances therefore means implanting different neural probes.

W02007042999 relates to a customizable multichanel microelectrode array with a modular planar microfabricated electrode array attached to a carrier and a high density of recording and/or stimulation electrode sites disposed thereon.

There is still a need in the art for a neural probe comprising one or more microelectrodes able to modulate their selectivity or impedance during the course of an experiment, which can run over several days or weeks.

### Summary of the invention

It is an object of embodiments of the present invention to provide alternative apparatus or methods for enabling the modulation of a microelectrode selectivity and/or impedance.

It is an advantage of embodiments of the present invention that good microelectrode recording capabilities can be obtained, also over extended periods.

It is a further advantage of embodiments of the present invention that the size and/or shape of a microelectrode can be modulated so as to enable the stimulation and/or recording of a different or changing neural area e.g. at the level of a single neuron or at the level of neighbouring neurons.

It is a further advantage of embodiments of the present invention that good functional exploration of cerebral tissue can be performed.

It is a further advantage of embodiments of the present invention that good functionally defined localization of electrical stimulation sites can be achieved.

It is a further advantage of embodiments of the present invention that good functional exploration of cerebral regions can be achieved.

It is a further advantage of embodiments of the present invention that the tracking of cerebral regions functionally connected with a recording site is enabled.

The above objective is accomplished by a method and device according to the present invention.

Electrical stimulation of biological tissues (in particular brain tissues) is strongly dependent on the characteristics of the microelectrode. The present invention discloses a way to obtain different configurations of a stimulation (and/or recording) microelectrode preferably using electronic switching integrated on the stimulation probe. By designing microelectrodes containing multiple, electrically independent segments, these segments can combine to obtain diverse microelectrode topologies. This allows the control of the microelectrode size and microelectrode shape.

In electrical recording this can be used to control microelectrode size and therefore vary the selectivity and impedance of microelectrodes during use or an experiment.

For example, by configuring the microelectrode topology electronically it becomes possible to do it during an experiment and to reconfigure it as the experiment progresses. In embodiments, it is also possible to perform this independently on multiple microelectrodes on the same probe.

In embodiments, instead of switching microelectrodes completely on (i.e. with ideally zero impedance between the microelectrode and the remaining circuitry) some additional control may be added (for instance voltage control in stimulation) in order to obtain a wider variety of electrical patterns from a given set of microelectrodes.

In a first aspect, the present invention relates to a neural probe system comprising:
● a carrier,
● at least one planar microelectrode attached to the carrier, said planar microelectrode comprising a set of two or more conductive segments, said set being preferably confined in an area of from 15 µm² to 8000 µm², and
● electrical connections assuring that at least two sub-sets of conductive segments within said set are individually switchable to the same input and/or output line so that the surface area and/or the shape of the microelectrode electrically connected to said line can be varied.

When not switched on to the same line, the conductive segments are electrically isolated from each other on the carrier.

In a second aspect, the present invention relates to a neural probe system comprising:
● a carrier,
● at least one planar microelectrode attached to the carrier, said planar microelectrode comprising a set of two or more conductive segments, said set being preferably confined in an area of from 15 µm² to 8000 µm², and
● electrical connections assuring that at least one sub-sets out of at least two sub-sets of conductive segments within said set is switchable to two or more lines selected from input and output lines.

In a third aspect the present invention relates to a method of recording neural activity via a neural probe according to any one of the preceding claims, said method comprising the step varying the size and/or the shape of said at least one planar microelectrode during said recording.

In a fourth aspect, the present invention relates to a method of recording neural activity via a neural probe according to the first aspect , wherein a stimulation step with a first subset is performed and a recording step with a second subset is performed.

Particular and preferred aspects of the invention are set out in the accompanying independent and dependent claims. Features from the dependent claims may be combined with features of the independent claims and with features of other dependent claims as appropriate and not merely as explicitly set out in the claims.

Although there has been constant improvement, change and evolution of devices in this field, the present concepts are believed to represent substantial new and novel improvements, including departures from prior practices, resulting in the provision of more efficient, stable and reliable devices of this nature.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### Brief description of the drawings

Fig. 1 is a diagrammatic illustration of a neural probe according to an embodiment of the present invention, an enlarged portion of said neural probe showing microelectrodes, and an enlarged portion of the electrical connections between the microelectrode segments and input/output lines in the case where each segment of a microelectrode is connected to the same input/output line.
Fig. 2 is a diagrammatic illustration of a microelectrode as used in embodiments of the present invention, wherein said microelectrode is shown to increase in size from left to right.
Fig. 3 is a diagrammatic illustration of a microelectrode as used in embodiments of the present invention, wherein said microelectrode is shown to change shape from left to right.
Fig. 4 is a diagrammatic illustration of a neural probe according to another embodiment of the present invention, an enlarged portion of said neural probe showing microelectrodes, and an enlarged portion of the electrical connections between the microelectrodes and input/output lines in the case where each segment of a microelectrode is connected to the same input/output line and wherein each segments of a microelectrode is also connected to the input/output line of all other microelectrodes.

In the different figures, the same reference signs refer to the same or analogous elements.

### Description of illustrative embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second, third and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Similarly, it is to be noticed that the term "coupled" should not be interpreted as being restricted to direct connections only. The terms "coupled" and "connected", along with their derivatives, may be used. It should be understood that these terms are not intended as synonyms for each other. Thus, the scope of the expression "a device A coupled to a device B" should not be limited to devices or systems wherein an output of device A is directly connected to an input of device B. It means that there exists a path between an output of A and an input of B which may be a path including other devices or means. "Coupled" may mean that two or more elements are either in direct physical or electrical contact or that two or more elements are not in direct contact with each other but yet still co-operate or interact with each other.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

As used herein and unless provided otherwise, the term *"switchable to"* when referring to a conductive segment means that a connection can be established (at one or more different potentials) or interrupted between said conductive segment and the element to which is said to be switchable.

In the description provided herein, numerous specific details are set forth. However, it is understood that embodiments of the invention may be practiced without these specific details. In other instances, well-known methods, structures and techniques have not been shown in detail in order not to obscure an understanding of this description.

The invention will now be described by a detailed description of several embodiments of the invention.
● Unless provided otherwise, the following embodiments are embodiments of the first and the second aspect of the present invention.

The microelectrode is designed as a set of conductive segments. The segments are electrically isolated from each other when not switched on to the same line. In an embodiment, each conductive segment or each sub-set of conductive segments may be independently switchable (e.g. between on and off or between on, off and one or more intermediate potentials). By grouping different segments together (i.e. connecting them to the same stimulation or recording circuitry), a specific microelectrode topology can be obtained. Hence, the shape or size of the microelectrode can be varied.

In an embodiment, at least one of said conductive segments is switchable to an input and/or output line via a passive or active circuit for adapting (or setting or adjusting) the potential of said segment.

In an embodiment, at least one of said at least two sub-sets is switchable to an input and/or output line via a passive or active circuit for adapting (or setting or adjusting) the potential of said sub-set.

In an embodiment, each of said at least two sub-sets of conductive segments are switchable to said same input and/or output line via a corresponding number of passive or active circuit for individually adapting (or setting or adjusting) the potential of said sub-sets.

The presence of a passive or active circuit for adapting the potential of a conductive segment is advantageous as it permits, in addition to the switching on and off of said segment to a line, to set the potential of the segment to a potential intermediate between fully on and fully off. For instance, a gradient of potential could be set in an electrode by setting a different potential for different subsets of segments. In other words, in addition to permitting a change of shape and/or size of the electrode, creation of a potential gradient within said electrode is made possible as well.

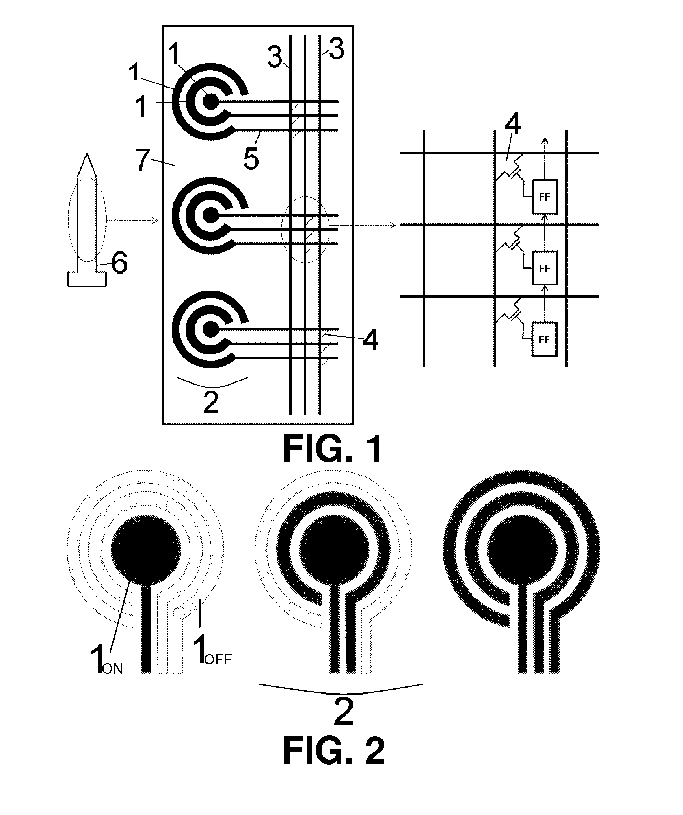

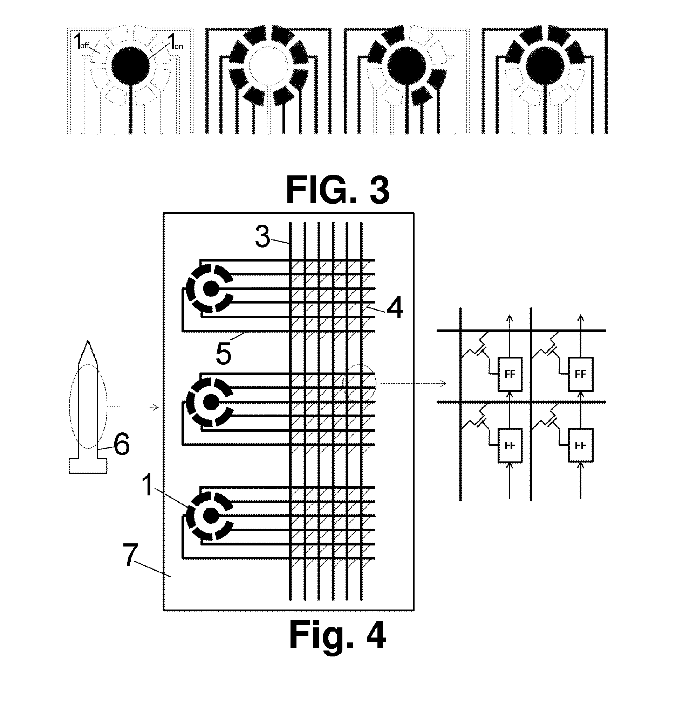

Fig. 2 and 3 exemplify two different designs and some of the possible configurations (black means active).

Embodiments of the neural probe systems of the first aspect permit on-the-fly change of the microelectrode shape or size. This allows the impedance and/or the selectivity of the microelectrode to be varied during the microelectrode use. This permits to adapt the microelectrode to a changing neuronal/microelectrode interface for instance.

In an embodiment, said subsets are individual segments. This is advantageous because it permits a finer tailoring of the microelectrode shape and/or size.

The carrier provides structural support.

In an embodiment, the carrier is made of a polymeric material such as a flexible polymeric material. Examples of such materials are polyimide or silicone.

In embodiments, the neural probe can comprise electrical connections (e.g. conductive interconnects) disposed between layers of dielectrics which insulate the electrical connections on top and bottom sides. In embodiments, at least some of these electrical connections terminate with a conductive segment of a microelectrode somewhere along the body of the neural probe and/or with bond pads for electrical connection to external instrumentation on the proximal end.

The electrical connections may be made of metal (e.g. gold or platinium). In embodiments, the dielectric may be a polymer (e.g. polyimide, parylene or PDMS). In another embodiment, the electrical connections may be made of metal or polysilicon insulated with inorganic dielectrics (such as Si₃N₄ or SiO₂) and/or polymers. In another embodiment, the electrical connections are made of polysilicon insulated with inorganic dielectrics that are supported by a silicon substrate. In another embodiment, the electrical connections maybe made above CMOS circuitry (used for example to obtain the switching of the electrode segments to the various input or output lines), using various methods for CMOS post-processing known to those skilled in the art of microsystem fabrication processes. In yet another embodiment, the neural probe system may be either a silicon or polymer-based structure with one or more microelectrodes, electrical connections and bond pads.

In embodiments, microelectrode segments and/or bond pads may be formed by opening apertures through a top dielectric and depositing and patterning metal (e.g. iridium, iridium oxide, platinum, gold).

Fabrication techniques for the segmented microelectrodes used in embodiments of the present invention generally do not depart from general manufacture techniques known from the person skilled in the art for fabricating non-segmented microelectrodes.

In embodiments of the present invention, multiple layers of electrical connections, each layer being separated by a layer of dielectric may be provided. This permits more densely packed electrical connections in a given width.

Producing a segmented microelectrode on silicon dioxide, silicon nitride, polyimide or parylene carrier may make use of a single step process involving etching an aperture through the top dielectric to expose a metal site below. In such cases, the microelectrode segment is contiguous with the electrical connections and the result is a microelectrode segment that is recessed within the top dielectric.

Microelectrode segments may also be formed using a three step process. First, a small site aperture may be etched through a top dielectric using reactive ion etching. The recess may next be filled by electroplating metal (e.g. gold, platinum) through a photolithographically defined mask. Finally, metal may be deposited and the segment may be formed using a conventional lift-off process.

In embodiments, when the carrier includes parylene, inorganic dielectrics, and/or silicon, a neural probe can be assembled that is comprised of the one or more segmented microelectrodes which will interface with neural tissue, and a foldable polyimide cable to transfer signals to/from the array. This polyimide cable may have bond pads on both its distal (for connecting to the segmented microelectrode) and proximal (for connecting to external instrumentation) ends. The microelectrode(s) and the cable can be coupled by methods known to those of ordinary skill in the art. In embodiments, exposed connections can be insulated by molding the device into a polymeric (e.g. silicone) structure that will also serve as the carrier.

As described herein, bond pads may be provided at the proximal end of the neural probe to provide a method for electrically contacting the array so that recorded signals can be accessed and/or so that stimuli may be provided. These pads can be bonded to a connector assembly (typically a form of printed-circuit board with or without on-board integrated circuits and a connector), or they can be connected directly to an Application Specific Integrated Circuit (ASIC). An example of an application for the latter case would be a multiplexer chip to reduce lead count, or a buffer amplifier to reduce signal loss over long leads.

In an embodiment, said electrical connections comprise interconnects between said segment(s) and said line, and switching devices (e.g. metastable switching devices or passive or active circuits for adapting the potential of said segment(s)) for switching said interconnects to said line.

In an embodiment, said switching device is a bistable switching device.

This enables the switching of segments on and off.

In an embodiment, said switching device may controlled electronically (e.g. the switching device is a flip flop). By configuring the microelectrode topology electronically it becomes possible to do it during use or an experiment and to reconfigure it as the experiment progresses. It is also possible to perform this independently on multiple microelectrodes on the same carrier.

In an embodiment, at least one of said subset (typically a segment) is linked to two or more different lines by a corresponding number of switches. This permits to perform signal triangulation (in recording mode) or differential stimulation.

In an embodiment, the number of microelectrodes equals the number of lines.

In an embodiment of the first aspect, at least a first of said at least two sub-sets of conductive segments within said set is switchable to a first input or output line and a second of said at least two sub-sets of conductive segments within said set is switchable to a second input or ouput line, different from said first input or output line. This permits differential operation of both sub-sets.

In an embodiment, at least an input line and an output line are present and at least one of said subset is switchable to said input line and at least another of said subset is switchable to said output line. This permits the performance of a stimulation step with said first subset while a recording step is performed with a second subset.

In an embodiment, said set may have an aspect ratio of from 1 to 3, preferably of from 1 to 2, more preferably of from 1 to 1.5 and most preferably of 1. This corresponds to the aspect ratio of a typical neural cell.

In an embodiment, said set may comprise circular and/or circular arc shaped segments. This enables a better fit of the shape of a neural cell.

In an embodiment, at least two of said circular arc shaped segments may be comprised in the same circle. This permits to encompass the shape of a globally circular neural cell.

In an embodiment, the geometrical centres of said circular shaped segments and/or the geometrical centres of the circles comprising said circular arc shaped segments may be confounded. This permits to encompass the shape of a globally circular neural cell.
In an embodiment, said set may have a circular or circular arc shape.

This permits to encompass the shape of a globally circular neural cell.

In an embodiment, the circle comprising said circular or circular arc shape has a diameter of from 5 to 100 µm. This corresponds to the typical size range of a neural cell.

In an embodiment, the number of segments is lower than or equal to 20. Although larger numbers are possible, it is usually sufficient to use 20 or less and even between 2 and 10 segments.

In an embodiment, at least two segments may have each independently a circular or a circular arc shape, the geometrical centers of said circular shaped segments and/or the geometrical center of the circles comprising said circular arc shaped segments may be confounded and said circular shaped segments and/or said circles comprising said circular arc shaped segments may have different diameters. This is the situation of for instance Fig. 2 and 3. This permits the size of the microelectrode to be modified while keeping a globally round like shape.

In an embodiment, more than one microelectrode as described in any embodiments is present on said carrier.
Fig. 1 shows a sketch exemplifying a possible implementation for switchable microelectrodes 2 as described in any embodiments of the first aspect of the present invention. The microelectrodes 2 are attached to a carrier 7 which is part of a neural probe 6. Concentric segments 1 are used to enable varying the effective microelectrode 2 size. In this case, all segments 1 of a given microelectrode 2 can be switched to the same line 3 and they are as many output/input lines as there are microelectrodes. The segments 1 are linked to said lines 3 via interconnects 5 and switches 4. In the embodiment of Fig. 1, the switch 4 comprises an electronically addressable flip flop FF.
Fig. 2 shows a microelectrode 2 composed of a set of three segments 1. At the left side of Fig. 2, the microelectrode 2 has a relatively small surface area due to only a single circular segment 1 being turned on. The central picture of Fig. 2 shows this same microelectrode 2 having two concentric and adjacent segments 1 turned on. As a result, this microelectrode 2 has a larger active surface area than in its state represented on the left side of Fig. 2. On the right side of Fig. 2, all segments 1 are turned on providing to the microelectrode 2 its largest size.
Fig. 3 shows a microelectrode 2 composed of a set of segments 1. At the left side of Fig. 3, the microelectrode 2 has a relatively small surface area due to only a single circular segment 1 being turned on. The second picture from the left shows that by switching off the central segment but switching on the segments 1 at the periphery, a microelectrode 2 of larger diameter but of hollow shape can be obtained. The third and fourth pictures from the left show other possible shapes obtainable by switching on or off specific segments 1 of the microelectrode 2. Not shown but off course also possible is a larger electrode obtained by switching on all segments 1 of the microelectrode 2.
Fig. 4 shows a neural probe 6 comprising three segmented microelectrodes, each having a variable shape. In this embodiment, a large number of switches 4 (comprising flip flops FF) and output/input lines 4 are present. The lines 3 are connected to the segments 1 via interconnects 5. This microelectrode 2 can be used in two ways. A first way is to switch all segments 1 of a same microelectrode 2 to the same input/output line 3, therefore enabling varying the effective microelectrode 2 shape. A second way is to switch segments 1 of a same microelectrode 2 to different output/input lines 3 to allow uses such as signal triangulation (in recording mode) or differential stimulation. Although only six output/input lines 3 are depicted, the number of lines is not limited. The same microelectrode 2 enables both uses.

In a second aspect, the present invention relates to a neural probe system comprising:
● a carrier,
● at least one planar microelectrode attached to the carrier, said planar microelectrode comprising a set of two or more conductive segments, said set being confined in an area of from 15 µm² to 8000 µm², and
● electrical connections assuring that at least one sub-sets of conductive segments within said set is switchable to two or more input and/or output lines by a corresponding number of switches.

In a third aspect, the present invention relates to a method of recording neural activity via a neural probe system according to any embodiment of the first aspect, said method comprising the step varying the size and/or the shape of said at least one planar microelectrode during said recording.

In a fourth aspect, the present invention relates to a method of recording neural activity via a neural probe system according to any embodiment of the first or second aspect wherein at least an input line and an output are present and at least one of said subset is switchable to said input line and at least another of said subset is switchable to said output line and wherein a stimulation step with a first subset is performed and a recording step with a second subset is performed.

Further aspect of the present invention relates to the use of a neural probe system according to any embodiment of the first or second aspect of the present invention for recording or stimulating a cell such as a neuron, a muscle cell or a pancreatic cell.

The recording of any of the methods disclosed above can be done in-vivo or ex-vivo on neural cells, muscle cells or pancreatic cells, for example. The method can be for therapeutic use or it can be for non-therapeutic use. In particular the use may be for brain function enhancement when there is no underlying pathological condition. Alternatively the neural probes may be used with patient's having Parkinson's, obsessive compulsive disorder (OCD), Alzheimer's and schizophrenia, for example. The neural probes according to the present invention can sense and stimulate individual cells electrically.
The neural probes can be used for understanding brain disorders and disease, or to study normal brain behaviour. The neural probes may be used in preoperative diagnostics prior to brain surgery for a variety of conditions.
The neural probes of the present invention may be used in combination, e.g. with functional magnetic resonance imaging (fMRI) to test certain behaviors, e.g. in animals.
Neural probes according to the present invention can be used to monitor mirror neurons. Mirror neurons fire when a person acts consciously, such as grasping an object, or if the subject watches somebody else perform the action. The same neurons fire when a person dreams about the action.
The neural probes of the present invention can be used a surgical guides to guide a surgeon to the focus, for example of epileptic seizures in a particular patient in order to render it inactive. Electrical sensing using the neural probes according to the present invention can be used to track a single cell actively or a group of cells. The neural probes of the present invention may also be used in cochlear implants. The neural probes according to the present invention can be used in a visual prosthesis.

It is to be understood that although preferred embodiments, specific constructions and configurations, as well as materials, have been discussed herein for devices according to the present invention, various changes or modifications in form and detail may be made. For example, steps may be added or deleted to methods described within the scope of the present invention.

## Claims

1. A neural probe system (6) comprising:
● a carrier (7),
● at least one planar microelectrode (2) attached to the carrier (7), said planar microelectrode comprising a set of two or more conductive segments (1), said set being confined in an area of from 15 µm² to 8000 µm², and
● electrical connections (4, 5) assuring that at least two sub-sets of conductive segments (1) within said set are individually switchable to a same input and/or output line (3) so that the surface area and/or the shape of the microelectrode (2) electrically connected to said line (3) can be varied.

2. The neural probe system (6) according to claim 1, wherein said subsets are individual segments (1).

3. The neural probe system (6) according to claim 1 or claim 2, wherein said electrical connections comprise:
● Interconnects (5) between said segment and said line (3), and
● switching devices (4) for switching said interconnects (5) to said line (3).

4. The neural probe system (6) according claim 3, wherein said switching device (4) is a bistable switching device (4).

5. The neural probe system (6) according to any one of the preceding claims, wherein at least one of said subset is linked to two or more different lines (3) by a corresponding number of switches (4).

6. The neural probe system (6) according to any one of the preceding claims, wherein the number of microelectrodes (2) equals the number of lines (5).

7. The neural probe system (6) according to any one of the preceding claims, wherein at least an input line (3) and an output line (3) are present and wherein at least one of said subset is switchable to said input line (3) and wherein at least another of said subset is switchable to said output line (3).

8. The neural probe system (6) according to any one of the previous claims, wherein said set has an aspect ratio of from 1 to 3, preferably of from 1 to 2, more preferably of from 1 to 1.5 and most preferably of 1.

9. The neural probe system (6) according to any one of the preceding claims, wherein said set comprises circular and/or circular arc shaped segments (1).

10. The neural probe system (6) according to claim 9, wherein at least two of said circular arc shaped segments (1) are comprised in the same circle.

11. The neural probe system (6) according to claim 9 or claim 10, wherein the geometrical centers of said circular shaped segments (1) and/or the geometrical center of the circles comprising said circular arc shaped segments (1) are confounded.

12. The neural probe system (6) according to any one of the preceding claims, wherein said set has a circular or circular arc shape.

13. The neural probe system (6) according to claim 12, wherein the circle comprising said circular or circular arc shape has a diameter of from 5 to 100 µm.

14. The neural probe system (6) according to any one of the preceding claims, wherein at least one of said conductive segments is switchable to an input and/or output line via a passive or active circuit for adapting the potential of said segment.

15. A non therapeutic method of recording neural activity via a neural probe system (6) according to any one of the preceding claims, said method comprising the step of varying the size and/or the shape of said at least one planar microelectrode (2) during said recording.

16. A non therapeutic method of recording neural activity via a neural probe system (6) according to claim 7, wherein a stimulation step with a first subset is performed and a recording step with a second subset is performed.

## Amended claims

### Amended claims in accordance with Rule 137(2) EPC.

**1.** A neural probe system (6) for recording or stimulating a cell such as a neuron, a muscle cell or a pancreatic cell, comprising:
• a carrier (7),
• at least one planar microelectrode (2) attached to the carrier (7), said planar microelectrode comprising a set of two or more conductive segments (1), said set being confined in an area of from 15 µm² to 8000 µm², and
• electrical connections (4, 5) assuring that at least two subsets of conductive segments (1) within said set are individually switchable to a same input and/or output line (3) so that the surface area and/or the shape of the microelectrode (2) electrically connected to said line (3) can be varied and wherein at least one of said subset is linked to two or more different lines (3) by a corresponding number of switches (4).

**2.** The neural probe system (6) according to claim 1, wherein said subsets are individual segments (1).

**3.** The neural probe system (6) according to claim 1 or claim 2, wherein said electrical connections comprise:
• Interconnects (5) between said segment and said line (3), and
• switching devices (4) for switching said interconnects (5) to said line (3).

**4.** The neural probe system (6) according claim 3, wherein said switching device (4) is a bistable switching device (4).

**5.** The neural probe system (6) according to any one of the preceding claims, wherein the number of microelectrodes (2) equals the number of lines (5).

**6.** The neural probe system (6) according to any one of the preceding claims, wherein at least an input line (3) and an output line (3) are present and wherein at least one of said subset is switchable to said input line (3) and wherein at least another of said subset is switchable to said output line (3).

**7.** The neural probe system (6) according to any one of the previous claims, wherein said set has an aspect ratio of from 1 to 3, preferably of from 1 to 2, more preferably of from 1 to 1.5 and most preferably of 1.

**8.** The neural probe system (6) according to any one of the preceding claims, wherein said set comprises circular and/or circular arc shaped segments (1).

**9.** The neural probe system (6) according to claim 8, wherein at least two of said circular arc shaped segments (1) are comprised in the same circle.

**10.** The neural probe system (6) according to claim 8 or claim 9, wherein the geometrical centers of said circular shaped segments (1) and/or the geometrical center of the circles comprising said circular arc shaped segments (1) are confounded.

**11.** The neural probe system (6) according to any one of the preceding claims, wherein said set has a circular or circular arc shape.

**12.** The neural probe system (6) according to claim 11, wherein the circle comprising said circular or circular arc shape has a diameter of from 5 to 100 µm.

**13.** The neural probe system (6) according to any one of the preceding claims, wherein at least one of said conductive segments is switchable to an input and/or output line via a passive or active circuit for adapting the potential of said segment.

**14.** A non therapeutic method of recording neural activity via a neural probe system (6) according to any one of the preceding claims, said method comprising the step of varying the size and/or the shape of said at least one planar microelectrode (2) during said recording.

**15.** A non therapeutic method of recording neural activity via a neural probe system (6) according to claim 6, wherein a stimulation step with a first subset is performed and a recording step with a second subset is performed.
